# EUROPEAN PATENT SPECIFICATION

(11) **EP 2 749 311 B1**
(45) Date of publication and mention of the grant of the patent: **15.04.2015**
(21) Application number: 13199489.9
(22) Date of filing: 23.12.2013
(51) Int. Cl.: A61M 25/00, A61M 27/00

(54) **Self-retentive and anti-reflux ureteral catheter**
Ureterkatheter mit Selbsthalterung und rückflussverhinderndem System
Cathéter urétral auto-rétentive avec anti-reflux

(30) Priority: 27.12.2012 ES 201232047
(43) Date of publication of application: 02.07.2014
(73) Proprietor: Fundacion Centro de Cirugia de Minima Invasion Jesus Uson, 10071 Caceres (ES)
(72) Inventor: Soria Galvez, Federico, 10071 CACERES (ES); Morcillo Martin, Esther, 10071 CACERES (ES); Sanchez Margallo, Francisco Miguel, 10071 CACERES (ES); Uson Gargallo, Jesus, 10071 CACERES (ES)
(74) Representative: Naranjo Marcos, Maria Antonia

(56) References cited:
- WO-A1-99/58083
- US-A1- 2003 199 986
- US-A1- 2004 059 279
- US-A1- 2007 032 880

## Description

### Aim of the invention

The invention, as stated in the heading of this descriptive document, is a new design for a self-retentive catheter which does not cause vesicoureteral reflux (anti-reflux).

Therefore, the purpose of this invention is a prosthesis which goes inside the ureter. It has three main features: Allowing the passive dilation of the upper ureter, which is necessary for carrying out endourologic techniques, and which in other circumstances serves as support for the ureter which has been operated on and requires time (3-6 weeks) to heal internally. Lastly, it solves cases of renal colic caused by kidney stone impaction. This design is aimed at resolving these problems in a single manoeuvre after freeing the prosthesis in the upper ureter. Thanks to its novel design and differences to current catheters, it doesn't have the same adverse effects as these devices usually have for patients who carry them.

As a result, the invention described falls in the category of implantable medical devices; specifically internal ureteral catheters.

### Background to the invention or State of the technique

The first ureteral catheter was created near the beginning of 1900 by Joaquin Albarran. In 1967, Zimskind was responsible for a significant step forward in the research and development of ureteral catheters. In his publication, the author describes the long-term use of silicon ureteral catheters inserted by cystoscopy. These catheters moved around easily because they were not anchored at either end. To reduce the movement, Gibbons designed a silicon catheter with several anchoring points along its length. However, these anchoring points significantly increased the outer diameter, were difficult to insert, and reduced the range of urinary flow.

The next design was aimed at preventing anterograde or retrograde displacement by creating an angle and forming a jay shape on both ends. In 1978, the authors Finney and Hepperlan described the double jay ureteral catheter. Later, to improve the double jay catheter, Mardis developed the double pigtail catheter, which had a lower tendency to move.

It is important to differentiate between ureteral catheters and ureteral stents, though both devices can be called stents. Ureteral catheters are usually plastic prostheses which go from the renal pelvis to the bladder (double jay or double pigtail catheters); whereas ureteral stents are usually made of metal, similar to vascular stents, and have the sole purpose of dilating a short section of the ureter in the manner of a bypass.

By definition, a ureteral catheter is a device made from synthetic polymer biomaterial designed to drain urine and remain between the renal pelvis and the bladder. The urinary system is an unstable environment chemically, with a supersaturation of mucous and crystalloid substances, which in the long term creates a significant problem in relation to the biocompatibility and biodurability of the catheter.

The ideal catheter should be designed to last, chemically stable, made from a biocompatible polymer, resistant to incrustation and infection, resist ureteral peristalsis, and move as little as possible. The catheter should ideally be easy to insert and comfortable for the patient, reacting minimally with the tissue. It should be highly elastic and enable the increase of urine flow from the moment it is inserted. In addition, it should be radiopaque and easily removed by endoscope or alternatively not require removal.

Another indispensable property of ureteral catheters is biomechanical memory to return to the dimensions it had before being physically distorted. This property is needed so that the catheter can be inserted using a guide, and then return to its jay shape configuration after the guide is removed. This memory allows the catheter to be retained without significant displacement. At present, all authors say that the ideal ureteral catheter does not exist, though there are a great variety of devices.

Double jay or pigtail catheters have become an integral part of contemporary urologic practice. As is the one protected by European Patent EP1685813. These catheters prevent or alleviate ureteral obstructions due to a wide range of intrinsic or extrinsic causes. These include: ureteral lithiasis, ureteral stenosis, congenital anomalies such as obstruction of the ureteropelvic junction, fibrosis or retroperitoneal tumours, neoplasms which extrinsically affect the upper ureter, or iatrogenic injuries. They are also widely used to provide urinary drainage following surgery of the upper ureter, independently of the procedure, or after an endourologic (ureteroscopy, lithiasis, endopyelotomy, endoureterotomy, etc.) or laparoscopic (pyeloplasty, ureteroneocystostomy, etc.) procedure, or by conventional surgery, as well as to dilate the upper ureter.

Payne and Ramsay observed that draining the bladder with a ureteral catheter caused a significant increase in intrarenal pressure, suggesting that vesicoureteral reflux contributes to increasing said pressure.

The ureter is then dilated after ureteral intubation. The ureteral catheter is used to therapeutically increase the ureteral diameter to examine the lumen and the passive exit of ureteral stones. This dilation can serve as a protective mechanism to minimise intrapelvic pressure resulting from the mechanical obstruction caused by the catheter.

The ureter is dilated due to the presence of the ureteral catheter because there is a reduction in peristalsis (physiological process consisting of radially symmetric contractions and relaxations which push urine from the ureter to the bladder), which is a result of the catheters impeding the coaptation of ureter walls. Mosly found a reduction of peristalsis in 80% of patients with a ureteral catheter. For this reason, the physiological flow of the ureter with a ureteral catheter would probably drain as a result of hydrostatic forces and gravity. Ureteral dilation and ineffective peristalsis remain for three weeks after the ureteral catheter is removed, meaning the ureterovesical junction is also dilated and we will encounter reflux during that time, prolonging the adverse effects of current ureteral catheters after they are removed.

Therefore, another known adverse effect in the ureter is vesicoureteral reflux. When a double jay ureteral catheter is inserted, the anti-reflux valve of the ureterovesical junction is interrupted and the ureteral meatus is kept open by the catheter. This vesicoureteral reflux only reaches the distal ureter, but during the physiological emptying of the bladder, pressure increases and reflux reaches the kidney. The reflux is produced in 80% of patients with a double jay catheter during emptying, and in 63% during vesical filling.

The range of adverse effects following ureteral catheterisation depends on many factors. These include the catheter material, the length of time it is in place, and presence or lack of an infection. Experimental studies show there is no reduction in renal filtration due to the catheter in the short term, and that there is renal dilation, inflammation changes, and vesicoureteral reflux, though all of these changes are temporary and disappear a few weeks after the ureteral catheter has been removed.

Therefore, it is proven that current commonly used double jay or double pigtail catheters lead to an increase in intrapelvic pressure, hydroureter, vesicoureteral reflux, and overall swelling of the ureteral wall with histological changes in the urothelium. After 1-3 weeks of ureteral catheterisation, the vesical mucus shows severe inflammation and ulceration with occasional metaplasia.

These effects cause significant morbidity in carriers, including: lumbar pain, suprapubic pain, dysuria, haematuria, microhaematuria, urinary infection, nocturia, frequent urination, tenesmus, urgency, incontinence, etc. The aetiology of this high percentage of morbidity associated to double jay ureteral catheters is not well known, but it has been shown that one of the determining factors is the increase of pressure in the renal pelvis due to vesicoureteral reflux caused by ureteral catheters, especially during vesical emptying. Another factor is irritation in the vesical trigone due to the intravesical section of the ureteral catheter. These two factors may be sufficient to explain the symptoms described above, given that the pain is a result of increased pelvic pressure, and the rest are symptoms associated to the irritation and erosion which can be caused by the vesical end of the ureteral catheters.

Therefore, the current conclusion is that in spite of new biomaterials, coatings, and designs which have been tested up to now, the ideal ureteral catheter does not yet exist, and we need to keep working to improve the design of these devices in order to reduce the adverse effects suffered by patients.

There have been countless advances in ureteral catheter design, but double jay catheters have not been displaced from the urologic repertoire. The following types are currently used:
- Spiral stents: such as the one protected by the patent with request number ES2075812, "Long lasting ureteral prosthesis". Their design is similar to metal ureteral stents but have a spiral shape and can be biodegradable. They are short tubular structures which allow urine to only pass through them, and instead of metal they are made from plastic polymers.
   Unlike the design presented in this document, spiral stents can only be used for extrinsic tumours and retroperitoneal fibrosis, dilating only the short section where they are placed. They don't allow for ablative treatment or post-surgery healing of the ureter.
- Tail stents: are designed to reduce vesical irritation. They are double jay catheters made from one thicker segment, and one narrower segment on the distal, vesical end. This configuration, in theory, results in a lesser vesical injury, as observed in the description of the American invention "Stent for implantation" (US 2007/0276466).
   Comprised in this category is D2 quoted in the prior art state report US pat 2007/0032880 MAEOA TORU on which the distal end of the catheter is kept inside the urine bladder.
   Same can be said concerning documents US2003/0199986 D4 MCWEENEY JOHN O [US] ET AL and WO99/58083 D3 TAYLOR WILLIAM, MCOOUGALL IAN quoted also in the prior art state report both inventions feature a catheter comprising a distal end with a structure that is held or retained inside the orine bladder , D4 features a structure which is fixed inside the distal end of the catheter while D3 features a ferromagnetic element inside said end.
- Dual-durometer catheters: they are made from different materials so that the catheter segment at the bladder is softer and presumably cause less irritation at this point. However, scientific publications say that in the short term there is no difference compared to classic ureteral catheters.
   Both tail stents and dual-durometer catheters have, due to their dual nature, with one end in the kidney and the other in the bladder, the same effects as double jay catheters. The design of the invention proposed in this document does not have these effects because it does not have the vesical end.
- Catheters with a modified distal end: this consists of modifying the end of the catheter which is anchored to the bladder with a loop made from a soft material than causes less vesical irritation. This is the case of the prosthesis described by the patent "Prosthesis having a sleeve valve", number WO 2003/011179.
   These devices, while they may cause less of an erosive injury to the bladder, still cause irritation, and they also cause vesicoureteral reflux. Both of these factors are absent in the design presented in this document.
   Prior art state document US 2004/059279 D1 MCWEENEY JOHN O [US] ET AL having a very similar design to the one featured in this invention particularly relating its distal end such end reaches the bladder once placed inside the ureter, in this invention the urine is transported by the distal end across the ureter/bladder junction and into the bladder.
- Anti-reflux double jay catheters: these are designed with a type of umbrella on the double jay vesical end so that at the time of emptying urine retrograde ureteral reflux becomes difficult. (WO 03/079930 A1; WO 03/053282 A1; WO 01/91668 A1).

Drugs have also been used to reduce the discomfort and adverse effects of ureteral catheters (reduce vesicoureteral reflux), with alpha-blockers the muscles of the intramural part of the ureter, vesical trigone, and prostate such as alfuzosin. However, in spite of improvements, there is no reduction in the consumption of analgesics. These catheters are steeped in these drugs, which are released slowly to alleviate the adverse effects resulting from the design of current ureteral catheters.

This approach can also be implemented in the proposed design, as the only requirement is to coat he device with the chosen drug. Both the design and the material allow the device to be coated with drugs, and the drugs to be released for as long as the catheter is in the patient.

### Explanation of the invention

The advantages of this catheter are described in the related claims.

In the attached designed sheets there is a drawing of the invention which is there to help understand the design. The drawing is for example purposes only and not an exhaustive description of the invention.

This new ureteral catheter is made up of 3 main elements which make up a single indivisible element:
The first is a hollow tube for introducing and fixing the device. It is radiopaque and jay shaped so it can be easily detected. It is placed in the renal pelvis to fix the catheter, making it self-retentive. This element has biomechanical memory, which makes it easier to insert, and once the insertion guide wire has been removed, it returns to its native jay shape, stopping the catheter from moving. At the same time, its hollow design allows the catheter to be placed under endoscopic or fluoroscopic control by inserting a guide wire along the same axis.
The second element of this catheter comprises a series of intertwined threads which have a variable length depending on the patient. This element does not have an internal channel, and urinary drainage takes place in a periprosthetic manner, around the catheter. Thus, there is an increase in the diameter of urinary evacuation in the vesical direction and the use of hollow tubular designs, which increase the external diameter of catheters and can be temporarily blocked by deposits of salts and detritus in urine, is avoided. Furthermore, this element fulfils the supportive purpose in the ureteral healing process and it allows the passive dilation of the ureteral segment in where it is located.

The intertwined design of the threads, combined with the absence of a metal centre in this segment, increase the flexibility of the catheter, which is good for the ureter.

The third element of the ureteral catheter comprises the continuation of the intertwined threads in the second section. In this case, the design consists of separating the threads and forming a double ellipse on two places which cross over at two points. This shape allows there not to be ureteral loops in the distal end of the catheter, which could cause alterations in urinary drainage, and it enables an increase in the urine's speed in this section.

The shape of the distal end follows a principle of fluid dynamics; specifically the Venturi effect, which says that if a fluid's flow is constant but the cross-section decreases, the velocity must increase through this section to satisfy the continuity equation such that *vₓ· Sₓ*= *v_{y} . S_{y}.* Thus, flow in motion along a ureter of diameter *S₁*, assuming that the fluid is incompressible and friction is negligible, will have a velocity *v₁*. When the conduct diameter changes to a variable *S₂*, where *S₂>S₁* at all times, velocity *v₂* will be less than *v₁*. Later, when there is another change in the ureter to a constant diameter *S₃,* where *S₃*>*S₁*>*S₂*, then it will be true that *v₃>v₂>v₁*.

Lastly, at the distal end of the catheter, the end of the third element, a radiopaque metal mark is added which allows us to see what point the prosthesis is reaching using fluoroscopic means.

A unique feature of the design of this ureteral catheter is that its positioning is exclusively intraurethral and at no point goes beyond the ureterovesical junction; that is, this catheter does not have an intravesical section. As such, the catheter does not cause vesicoureteral reflux and, since its distal end does not sit on the vesical neck, it avoids all the complications associated to current ureteral catheters (lumbar pain, suprapubic pain, dysuria, haematuria, microhaematuria, urinary infection, urinary incontinence, nocturia, frequent urination, tenesmus, urgency, incomplete vesical emptying, etc.).

It is important to highlight that this ureteral catheter design can be manufactured with non-resorbable material and need to be extracted at a later date, but it can also be made with biodegradable material at a urinary level, meaning that after performing its function as a catheter it would degrade in the ureter because of the urine, without the need of a second intervention to extract it.

Given all of the above and in light of the analysis of the current state of the technique, the advantages of this invention are clear to see. Below we will list some of the most important advantages. This list is not exhaustive.
- The catheter can be manufactured to have different lengths and with different materials according to the physiology of the patient or to the characteristics of the disease.
- It can be made from biodegradable materials which will be reabsorbed by the organism after the fulfilling the catheter function, meaning that a second intervention to remove the device is not required.
- Its distal end is not placed inside the bladder, so the adverse effects of current catheters which lack this feature are not present.
- Its central structure is highly elastic, which is good for the ureter in which it is inserted.
- It allows for increased urinary flow from the time that it is inserted.
- It is designed to resist incrustation, infection, and ureteral peristalsis, minimising the risk of displacement.
- Its unique design allows urine to flow normally from the kidney to the bladder, avoiding obstructions which can arise in current catheters.
- It is easy to manufacture.

### Description of the drawings

To complete the description of the invention which is the subject of this technical report, and better explain its characteristics, a design sheet is attached with different preferential ways of building the invention. These designs are not an exhaustive list.
Figure 1 shows a top view of the ureteral catheter, displaying the whole composition.

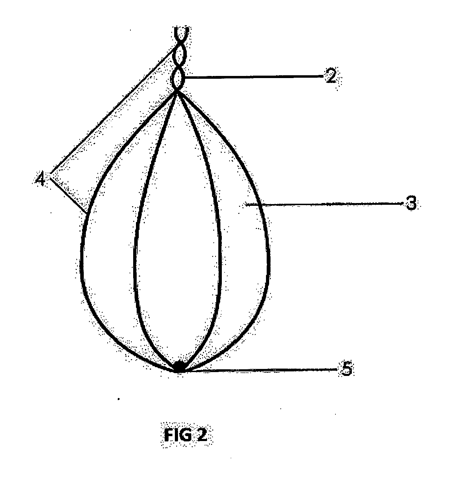

Figure 2 gives a detailed view of the distal end of the catheter which will be inside the ureter (U). Here it can be seen how the thread which makes up te catheter structure ends in a double ellipse shape.
Figure 3 shows the insertion method of the catheter through the ureter conduct, extending towards the bladder (v) and the ureter (U) up to the renal pelvis (R). Insertion is achieved with the help of a guide wire.
Figure 4 gives a graphic representation of a nephroureteral unit and of the bladder (V), with the self-retentive and anti-reflux ureteral catheter correctly placed in the ureter. The near end adopts the characteristic jay shape to remain fixed without the distal end going beyond the ureter (U) mouth in the bladder (V).

### Exposition of a preferential realisation of the invention

Based on the drawings which are attached to this technical report, below is a description for the preferential realisation of the invention by way of an example.

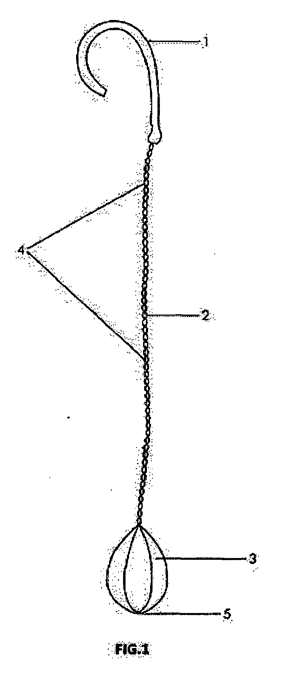

As can be seen in figure 1, the invention described in this document is a ureteral catheter made from three elements: the first, in the near end, is a hollow tube (1) which allows the catheter to be inserted and implanted; the second it the main body (2) of the catheter, made from intertwined threads (4) whose continuation (3) in a double ellipse configuration at the distal end of the device makes up the third element.

The materials that the invention can be made from are varied. Depending on surgical requirements, resorbable and biodegradable materials can be used, as well as materials which do not have these characteristics and would make the catheter last longer.

At the same time, the catheter's design means that it can be manufactured in different sizes and be adapted to required dimensions, depending on the patient's organic proportions.

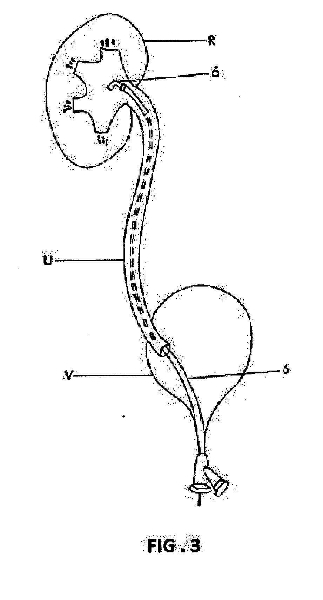

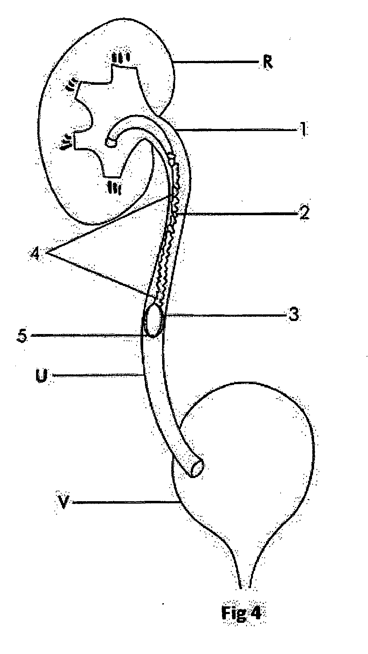

The first element is a radiopaque, jay-shaped, hollow tube (1) with biomechanical memory. Its hollow design enables the catheter to be introduced through the urethra to the renal pelvis (R) by endoscopic or fluoroscopic control by inserting a guide wire (6) along the same axis, as shown in figure 3. At the same time its biomechanical memory causes the hollow tube (4) to recover its jay shape after said guide wire (6) is released. The tube then becomes fixed to the renal pelvis (R) in a stable manner, as shown in figure 4.

The second element of the main body of the catheter (2) is a group of variable length intertwined threads (4). This design for the main body (1) does not feature a metal centre, thus increasing its flexibility. Urine is drained around the catheter.

Lastly, the third element is the continuation (3) of the intertwined threads (4) of the main body (2) of the invention. The threads separate to form a double ellipse on two plains which cross at two points. One of them, at the distal ends, includes a radiopaque metal mark (5) which, together with the hollow tube (1), allows the placement of the catheter inside the ureter (U) to be controlled by fluoroscope.

The design of the continuation (3) of the intertwined thread (4) in a double ellipse shape is based on a fluid dynamics principle called the Venturi principle, which states that a flow moving at a constant rate through a closed conduct, when passing through a smaller section, will decrease its pressure after passing through that area, and thus increase its speed. This principle is applied to the pass of urine around the catheter, allowing optimum drainage at higher speed.

This continuation (3) on the distal end of the catheter has been conceived and developed so that it stays inside the ureter (U) and at no time reaches the bladder (V).

## Claims

1. Self-retentive and anti-reflux ureteral catheter that comprises the parts described below: A jay shaped hollow tube (1) with biomechanical memory on its near end, which serves to insert and fix the device in the ureter; a main body (2) made of interwined threads (4) which facilitate urine drainage, with the whole catheter being shorter than the ureter (U); a distal end (3), the distal end remaining inside of the ureter (U) **characterized in that** said distal end (3) includes the end of the intertwined threads (4) of the main body (2), which separate to form a double ellipse which joins in two points, the most distal of which contains a radiopaque metal marker (5).

## Patentansprüche

1. Selbsthaltender und rückflussgesicherter Harnleiterkatheter, der unten genannte Teile umfasst; ein hohles Röhrchen in J-Form (1) mit System zur biomechanischen Restabilisierung (biomechanical memory) am proximalen Ende, welches zum Einführen und Befestigen der Vorrichtung im Harnleiter dient; ein Hauptkörper (2), bestehend aus miteinander verflochtenen Drähten (4), der die Harnableitung erleichtert, wobei der gesamte Katheter kürzer als der Harnleiter (U) ist; **dadurch gekennzeichnet, dass** besagtes distales Ende (3) im Innern des Harnleiters (U) verbleibt und das Ende der miteinander verflochtenen Drähte (4) des Hauptkörpers (2) einschließt, welche sich teilen, um eine doppelte Ellipse zu bilden, die an den zwei Punkten verbindet, von denen der distale eine strahlenundurchlässige Metallmarke (5) umfasst.

## Revendications

1. Cathéter urétéral auto-rétention et anti-reflux composé des deux parties décrites ci-dessous ; un tube creux en forme de J (1), avec mémoire biomécanique à son extrémité la plus proche, qui sert à insérer et à fixer le dispositif dans l'uretère, un corps principal (2) réalisé en fils entrelacés (4) qui facilite le drainage de l'urine, le cathéter complet étant plus court que l'uretère (U) **caractérisé par** ladite extrémité distale (3), reste à l'intérieur de l'uretère (U) et inclut l'extrémité des fils entrelacés (4) du corps principal (2) qui se sépare pour former une double ellipse qui unit les deux points, le plus distal possède une marque métallique (5) radio-opaque.
